# EUROPEAN PATENT APPLICATION

(11) **EP 1 447 074 A2**
(43) Date of publication of application: **18.08.2004**
(21) Application number: 04250638.6
(22) Date of filing: 06.02.2004
(51) Int. Cl.: A61K 7/027, A61K 7/50

(54) **Polymeric nanoparticles in consumer products**

(30) Priority: 12.02.2003 US 447217 P
(71) Applicant: ROHM AND HAAS COMPANY, Philadelphia, Pennsylvania 19106-2399 (US)
(72) Inventor: Lundquist, Eric Gustave, North Wales, Pennsylvania 19454 (US); Devonport, Wayne, Doylestown, Pennsylvania 18901 (US); Will, Joanne Patricia, Easton, Pennsylvania 18042 (US)
(74) Representative: Kent, Venetia Katherine

(57) **Abstract**

The present invention relates to crosslinked polymeric nanoparticles having a diameter of 1-10 nm comprising skin care ingredients and food ingredients.

## Description

### BACKGROUND OF THE INVENTION

The successful marketing of a consumer product depends on said product's ability to appeal to a consumer's sense of taste, smell and touch. For example, food products must have good taste and good mouth feel (i.e., a creamy, smooth texture) and personal care products should smell good and feel good in contact with the body along with delivering the promised effect. Those skilled in the art have a wide range of chemistries to impart good taste, good smells, good tactile properties and effectiveness to consumer products. However, the art is still in need of systems to more effectively deliver these useful chemistries. A plethora of patents address these problems. For example, US 6,013,271; 6,331,305; and 6,316,010 disclose the problems associated with skin care preparations such as ease of application, skin feel, and delivery of an effective amount of skin care ingredient. US 5,885,594; 6,436,459; and 6,432,465 disclose the problems associated with preparing tasty, low fat, nutritious snacks. The personal care art has attempted to solve the problems associated with delivering skin care active ingredients. Specifically, WO 01/43859 (Cognis) discloses that lotions, creams, and body cleansing preparations are improved by the addition of nanoscale polymers in the 10-500 nm size range. Nano-sized polymer particles (<100 nm) have extremely high surface areas, are transparent (under the wavelength of visible light), and are able to penetrate through membranes and barriers not normally penetrable by larger materials. Said, polymer nanoparticles have attracted increased attention over the past several years in a variety of fields including catalysis, coatings, medicine, electronics, and polymeric composites. Said nanoscale polymers disclosed in Cognis can be vinyl acetate/crotonic acid copolymers, vinyl pyrrolidone/vinyl acrylate copolymers, vinyl acetate/butyl maleate/isobornyl acrylate copolymers, uncrosslinked and polyol-crosslinked polyacrylic acids, acrylamidopropyl trimethyl ammonium chloride/acrylate copolymers, octyl acrylamide/methyl methacrylate/*tert*.-butylaminoethyl methacrylate/2-hydroxyl-propyl methacrylate copolymers, polyvinyl pyrrolidone, vinyl pyrrolidone/vinyl acetate copolymers, natural polymers such as guar, cellulose, starch and/or alginates and proteins, and protein derivatives.

EP 1,146,057 (Givaudan) discloses that olfactive molecules can be incorporated into nanoparticles in the 250 nm―500 nm size range. Said nanoparticles can comprise styrene and acrylate copolymers. They can be crosslinked.

Neither EP 1,146,057 or WO 01/43859 disclose the use of crosslinked polymer nanoparticles that are under 10 nm in size for use in consumer products.

Applicants have enhanced the art by providing a smaller crosslinked polymeric nanoparticle capable of carrying skin care ingredients and food ingredients. Said polymeric nanoparticle can be expected to be more efficacious due to its smaller size. Further, Applicants have met a long-felt need for a new line of carrier materials to deliver a wide variety of consumer product ingredients.

### SUMMARY OF THE INVENTION

The present invention relates to crosslinked polymeric nanoparticles having a diameter of 1-10 nm comprising skin care ingredients.

The present invention relates to crosslinked polymeric nanoparticles having a diameter of 1-10 nm comprising food ingredients.

The following terms and definitions have the following meanings herein:

The term "skin care "includes hair care.

The term "skin care ingredients" as used herein means abrasives, absorbents, aesthetic components such as fragrances, cleansing agents, pigments, colorings/colorants, essential oils, skin sensates, astringents, etc. (e.g., clove oil, menthol, camphor, eucalyptus oil, eugenol, menthyl lactate, witch hazel distillate), anti-acne agents, anti-caking agents, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, film formers or materials (e.g., polymers for aiding the film-forming properties and substantivity of the composition such as copolymers of eicosene and vinyl pyrrolidone), opacifying agents, pH adjusters, propellants, reducing agents, sequestrants, skin bleaching and lightening agents (e.g., hydroquinone, kojic acid, ascorbic acid, magnesium ascorbyl phosphate, ascorbyl glucosamine), skin conditioning agents (e.g., humectants, including emollients and occlusive agents), skin soothing and/or healing agents (e.g., panthenol, aloe vera, pantothenic aid, allantoin, bisabolol, and dipotassium glycyrrhizinate), antimicrobials such as triclosan, thickeners, surfactants, and vitamins.

The term "food ingredient "as used herein means any edible material used to impart one or more of the following properties to a food item: taste, texture, nutritional value, or sensory appeal.

### DETAILED DESCRIPTION OF THE INVENTION

The present invention relates to crosslinked polymeric nanoparticles having a diameter of 1-10 nm comprising skin care ingredients.

The present invention relates to crosslinked polymeric nanoparticles having a diameter of 1-10 nm comprising food ingredients.

Polymeric nanoparticles useful in the practice of the present invention can be formed from natural materials and synthetic materials and mixtures thereof.

Preferably the crosslinked polymeric nanoparticles are from 1-10 nm in diameter, more preferably 2 to 8 nm in diameter.

Natural materials useful in making the polymeric nanoparticles include, but are not limited to, sugars, starches, dextrans, polysaccharides, proteins, alginates, and cellulose. Sugars include, but are not limited to, glucose, fructose, sucrose, maltose, lactose, maltitol, sorbitol, mannitol, and xylitol. Starches include, but are not limited to, potato, corn, and wheat. Proteins include those proteins derived from animal or plant sources.

Synthetic materials useful in making the polymeric nanoparticles include, but are not limited to, those made from monomers of alkyl (meth)acrylates, alicyclic (meth)acrylates, (meth)acrylamides, vinyl acetates, alkenyl (meth)acrylates, aryl (meth)acrylates, alkylaryl (meth)acrylates, vinyl aromatic monomers, (meth)acrylic acid, and substituted ethylene monomers.

Exemplary alkyl (meth)acrylates useful in making the polymeric nanoparticles (PNPs) of the present invention include, for example, methyl methacrylate ("MMA"), methyl acrylate, ethyl acrylate, propyl methacrylate, butyl methacrylate ("BMA"), butyl acrylate ("BA"), IBMA, hexyl methacrylate, cyclohexyl methacrylate, cyclohexyl acrylate, cetyl methacrylate, stearyl methacrylate, 2-ethylhexyl acrylate ("EHA"), 2-ethylhexyl methacrylate, octyl methacrylate, decyl methacrylate, isodecyl methacrylate, undecyl methacrylate, dodecyl methacrylate, tridecyl methacrylate, tetradecyl methacrylate, pentadecyl methacrylate, hexadecyl methacrylate, heptadecyl methacrylate, octadecyl methacrylate, nonadecyl methacrylate, cosyl methacrylate, eicosyl methacrylate, lauryl methacrylate (LMA), and mixtures thereof.

Useful substituted alkyl (meth)acrylate monomers for making PNPs containing acid functional monomers may include those with one or more hydroxyl groups in the alkyl radical, such as hydroxyalkyl (meth)acrylate monomers having a substituted alkyl group selected from the group consisting of (C₂-C₆)alkyl, branched and unbranched alkyl groups. Likewise, where hydroxyl group containing monomers or monomers containing acetoacetoxy groups are used to make PNPs, acid functional monomers can be added to react with those groups.

Examples of these monomers are hydroxylalkyl (meth)acrylate 2-hydroxyethyl methacrylate ("HEMA"), 2-hydroxyethyl acrylate ("HEA"), 2-hydroxypropyl methacrylate, and acetoacetoxy methacrylate.
Other substituted (meth)acrylate monomers useful in the present invention may include silicon-containing monomers such as polymethylsiloxane diacrylate, γ-propyl tri(C₁-C₆) alkoxysilyl (meth)acrylate, γ-propyl tri(C₁-C₆) alkylsilyl (meth)acrylate, γ-propyl di(C₁-C₆) alkoxy (C₁-C₆)alkylsilyl (meth)acrylate, γ-propyl di(C₁-C₆)alkyl(C₁-C₆)alkoxysilyl (meth)acrylate, vinyl tri(C₁-C₆)alkoxysilyl (meth)acrylate, vinyl di(C₁-C₆)alkoxy (C₁-C₆)alkylsilyl (meth)acrylate, vinyl (C₁-C₆) alkoxydi (C₁-C₆)alkylsilyl (meth)acrylate, vinyl tri(C₁-C₆)alkylsilyl (meth)acrylate, and mixtures thereof.

Vinylaromatic monomers useful as unsaturated monomers in the present invention may include styrene ("STY"), α-methylstyrene, vinyltoluene, *p*-methylstyrene, ethylvinylbenzene, vinylnaphthalene, vinylxylenes, and mixtures thereof.

Exemplary ethylenic and substituted ethylene monomers useful as unsaturated monomers in the present invention may include cyclopentadiene, allylic monomers, vinyl acetate, vinyl formamide, vinyl chloride, vinyl fluoride, vinyl bromide, vinylidene chloride, vinylidene fluoride, and vinylidene bromide. However, it is preferable to use those monomers which can be polymerized using initiators commonly used to polymerize (meth)acrylate monomers, e.g. dibenzoyl peroxide.

Said polymers contain crosslinkers such as di-, tri-, tetra-, or higher multifunctional ethylenically unsaturated monomers such as ethyleneglycol diacrylate, trimethylolpropane triacrylate, allyl methacrylate (ALMA), ethyleneglycol dimethacrylate (EGDMA), DEGDMA, propyleneglycol dimethacrylate, propyleneglycol diacrylate, TMPTMA, 2,2-dimethylpropane-1,3-diacrylate, 1,3-butylene glycol diacrylate, 1,3-butylene glycol dimethacrylate, 1,4-butanediol diacrylate, diethylene glycol diacrylate, diethylene glycol dimethacrylate, 1,6-hexanediol diacrylate, 1-6-hexanediol dimethacrylate, tripropylene glycol diacrylate, triethylene glycol dimethacrylate, tetraethylene glycol diacrylate, polyethylene glycol 200 diacrylate, tetraethylene glycol dimethacrylate, polyethylene glycol dimethacrylate, ethoxylated bisphenol A diacrylate, ethoxylated bisphenol A dimethacrylate, polyethylene glycol 600 dimethacrylate, poly(butanediol) diacrylate, pentaerythritol triacrylate, trimethylolpropane triethoxy triacrylate, glyceryl propoxy triacrylate, pentaerythriotal tetraacrylate (PETTA), pentaerythritol tetramethacrylate (PETMA), and mixtures thereof. Other crosslinkers useful in the present invention may include DVB, vinyl urethanes, diallyl ethers, diallyl esters, vinyl polyesters, trivinylbenzene, divinyltoluene, divinylpyridine, divinylnaphthalene and divinylxylene, bisacrylamide, triallyl cyanurate, diethyleneglycol divinyl ether, and trivinylcyclohexane.

Suitable multiethylenically unsaturated functionalized polymers for use as crosslinkers in the present invention include, but are not limited to, acrylate and styrene functionalized homo- and co-polymers derived from the reaction of an appropriately functionalized vinyl monomer with a polymer comprising of, but not limited to, poly alcohols, such as poly(ethylene glycol), poly(proylene glycol), hydrolyzed and partially hydrolyzed poly(vinyl alcohol); poly(siloxane), such as poly(dimethylsiloxane); poly(hydroxyethyl acrylate); poly(hydroxypropyl acrylate); poly(hydroxy styrene); starch; saccharides such as glucose, dextrose, fructose, dextran, cyclodextrin, cellulose, chitin, chitosan. Other polymersd sutiable for use as crosslinkers include, but are not limited to, functionalizable polymers such as: poly(allylamine); polyacrylic acid; poly(acryloyl chloride); polyalanine; poly(aminostyrene); polyaniline; poly(bromostyrene); polybutadiene; polyamides and polyesters such as polyhydroxyalkanoates; polycaprolactone, polycaprolactone diol, polyethyleneterephthalate, nylon-6,6; polyethyleneimine; poly(furfuryl alcohol); poly(glycolide); poly(lactide); polylactic acid; poly(itaconic acid); poly(maleic acid); poly(maleic anhydride); poly(vinylamine); poly(vinyl chloride); hydroxy, carboxylic acid, halogen, and amino functionalized dendrimers or hyperbranched polymers such as poly(amidoamines); poly(benzylethers), poly(alkylimines). The aforementioned crosslinkers preferably have a molecular weight less than or equal to 10,000 Daltons.

The crosslinked PNPs of the present invention may be formed by polymerization techniques known to those skilled in the art. These techniques include, but are not limited to, condensation polymerization, cationic polymerization, ring opening metathesis polymerization, anionic polymerization and free radical polymerization. Free radical polymerization is preferred. Initiators useful in the free radical polymerization of the present invention include, for example, one or more of: peroxyesters, dialkylperoxides, alkylhydroperoxides, persulfates, azoinitiators, redox initiators, and the like. Other useful free radical initiators include, but are not limited to: benzoyl peroxide, t-butyl peroctoate, t-amyl peroxypivalate, cumene hydroperoxide, and azo compounds such as azoisobutyronitrile and 2,2'-azo*bis* (2-methylbutanenitrile). Preferably, the free radical initiator is t-amyl peroxypivalate. The amount of the free radical initiator used is typically from 0.05 to 10% by weight, based on the weight of total monomer.

### Skin Care Ingredients Useful in the Practice of the Present Invention

Those skilled in the art are knowledgeable about skin care ingredients. The CTFA Cosmetic Ingredient Handbook is one of many useful sources for such information. See also, Balsam & Sagarin, Cosmetics: Science and Technology, 2^{nd} Edit., Wiley InterScience (Balsam & Sagarin). Skin care ingredients useful in the practice of the present invention include, but are not limited to, desquamation actives such as sulfhydryl compounds and zwitterionic surfactants and salicylic acid; anti-acne agents such as retinoid compounds, benzoyl peroxide and salicylic acid; UVA and UVB sun blockers, anti-wrinkle actives such as hydroxyacids (alpha or beta), salicylic acid, glycolic acid, retinoids and skin peel agents such as phenol; fat and water soluble vitamins such as E and B₃ and C; flavonoids; tanning actives; anti-inflammatories; pigments, antibacterials such as triclosan; soothing agents such as bisabolol and sunscreen activities. Further, thickeners, preservatives, emollients, cleansing agents, humectants, and surfactants are useful in the practice of the present invention.

Thickeners or rheology modifiers are useful to increase the viscosity and to maintain the viscosity at required levels under specified processing conditions and end use situations. For example, thickeners are used in hair fixture formulations not only for viscosity improvement and control, but also for protective colloidal action and for improvement of pigment suspension, leveling and flow. In addition, thickeners can emulsify, disperse and stabilize latex ingredients. They can also be film formers. Thickeners are also utilized for their suspending properties, as well as to improve product appeal, i.e., smooth and silky sensations when in contact with the skin.

Well-known thickeners include, but are not limited to, natural products such as the alginates, casein, gum karaya, locust bean gum and gum tragacanth, and modified natural products such as the cellulosics, including methyl cellulose, hydroxyethyl cellulose and hydroxypropylmethyl cellulose. Totally synthetic thickeners are also available, such as carboxy vinyl ether copolymers, acrylic polymers, and maleic anhydride/styrene copolymers.

Preservatives are used to prevent microbial contamination of the skin care product itself, as well as to provide an antimicrobial effect for the consumer. Preservatives used in skin care formulations include, but are not limited to, isothiazolones, triclosan, cetyl pyridimonium chloride, and methyl and propyl paraben.

Suitable emollients for use in the present invention include, but are not limited to, isostearic acid derivatives, isopropyl palmitate, lanolin oil, diisopropyl dimerate, maleated soybean oil, octyl palmitate, isopropyl isostearate, cetyl lactate, cetyl ricinoleate, tocopheryl acetate, acetylated lanolin alcohol, cetyl acetate, phenyl trimethicone, glyceryl oleate, tocopheryl linoleate, wheat germ glycerides, arachidyl propionate, myristyl lactate, decyl oleate, propylene glycol ricinoleate, isopropyl lanolate, pentaerythrityl tetrastearate, neopentylglycol dicaprylate/dicaprate, hydrogenated cocoglycerides, isononyl isononanoate, isotridecyl isononanoate, myristal myristate, triisocetyl citrate, cetyl alcohol, octyl dodecanol, oleyl alcohol, panthenol, lanolin alcohol, linoleic acid, linolenic acid, sucrose esters of fatty acids, octyl hydroxystearate and mixtures thereof. Examples of other suitable emollients described in the Cosmetic Bench Reference, pp. 1.19-1.22 (1996) and Balsam & Sagarin.

Non-polar emollients are especially useful. "Non-polar emollient," as used herein, means any emollient emulsifier possessing no permanent electric dipole moments. Suitable non-polar emollients include but are not limited to, esters and linear or branched chained hydrocarbons, examples of which include isononyl isononanoate, isopropyl isostearate, octyl hydroxystearate, diisopropyl dimerate, lanolin oil, octyl palmitate, isopropyl palmitate, paraffins, isoparrafins, acetylated lanolin, sucrose fatty acid esters, isopropyl myristate, isopropyl stearate, mineral oil, silicone oils, dimethicone, allantoin, isohexadecane, isododecane, petrolatum, and mixtures thereof. Suitable oil emollients for use in the compositions of the present invention include esters, triglycerides, hydrocarbons, and silicones such as dimethicones, flurosilicones, polyalkyl siloxanes, organosiloxanes, and ammonium modified silicones. These can be a single material or a mixture of one or more materials.

Oils can act as emollients and can also impart viscosity, tackiness, and drag properties to cosmetic compositions such as lipsticks. Examples of suitable oils include caprylic triglycerides; capric triglyceride; isostearic triglyceride; adipic triglyceride; propylene glycol myristyl acetate; lanolin; lanolin oil; polybutene; isopropyl palmitate; isopropyl myristate; isopropyl isostearate; diethyl sebacate; diisopropyl adipate; tocopheryl acetate; tocopheryl linoleate; hexadecyl stearate; ethyl lactate; cetyl; oleate; cetyl ricinoleate; oleyl alcohol; hexadecyl alcohol; octyl hyroxystearate; octyl dodecanol; wheat germ oil; hydrogenated vegetable oils; castor oil; petrolatum; modified lanolins; branched-chain hydrocarbons, alcohols and esters, corn oil; cottonseed oil; olive oil; palm kernel oil; rapeseed oil; safflower oil; jojoba oil, evening primrose oil; avocado oil mineral oil, sheabutter, octylpalmitate, maleated soybean oil, glycerol trioctanoate, diisopropyl dimerate, and volatile and non-volatile silicone oils including phenyl trimethicone.
The preferred oils for use herein are acetylglycerides, octanoates, and decanoates of alcohols and polyalcohols, such as those of glycol and glycerol, the ricinoleates of alcohols and polyalcohols such as cetyl ricinoleate, PG-3 diisosterate, polyglycerol ethers, polyglycerol esters, caprylic triglycerides, capric triglycerides, isostearic triglyceride, adipic triglyceride, phenyl trimethicone, lanolin oil, polybutene, isopropyl palmitate, isopropyl isostearate, cetyl ricinoleate, octyl dodecanol, oleyl alcohol, hydrogenated vegetable oils, castor oil, modified lanolins, octyl palmitate, lanolin oil, maleated soybean oil, cetyl ricinoleate, glyceryl trioctanoate, diisopropyl dimerate, synthetic lanolin derivatives and branched chain alcohols sucrose esters of fatty acids, octyl hydroxystearate, and mixtures thereof.

Humectants useful in the practice of the present invention include, but are not limited to, sorbitol, propylene glycol, butylene glycol, hexylene glycol, ethoxylated glucose derivatives, hexanetriol derivatives, glycerine, water soluble polyglyceryl methacrylate lubricants, and panthenols.

Surfactants useful in the practice of the present invention include, but are not limited to, those that are especially well-tolerated by the skin such as fatty alcohol polyglycol ether sulfates, monoglyceride sulfates, mono- and/or dialkyl sulfosuccinates, fatty acid isethionates, fatty acid sarcosinates, fatty acid taurides, fatty acid glutamates, α-olefin sulfonates, ethercarboxylic acids, alkyl oligoglucosides, fatty acid glucamides, alkylamidobetaines, amphoacetates, and/or protein-fatty acid condensates, the latter being preferably based on wheat proteins.

### Food Ingredients Useful in the Practice of the Present Invention

Those skilled in the food industry are familiar with the ingredients used to impart taste, texture, nutritional value, or sensory appeal to food. Food ingredients useful in the practice of the present invention include, but are not limited to, flavorings; vitamins (fat and water soluble); fats; fat substitutes such as acyl glycerol and polyol fatty acid polyesters; proteins; fiber; carbohydrates such as sugars and starches; and processing aids.

Non-limiting examples of useful food ingredients are disclosed in U.S. 2002/0015760; U.S. 5,885,594; and U.S. 2002/0106438A1.

### Preparing the skin care ingredient or food ingredient/PNP blend

The skin care ingredient or the food ingredient is blended with the 1-10 nm diameter crosslinked polymeric nanoparticle. Said crosslinked polymeric nanoparticle is now said to be carrying the skin care or food ingredient. The following designation is used herein to describe said ingredient and polymeric nanoparticle (PNP) blend: name of ingredient/polymeric nanoparticle (PNP) blend. Said polymeric nanoparticle carrying said ingredient can now be used to formulate the food or skin care product. For example, a butter flavor/polymeric nanoparticle (PNP) blend can be sprayed onto the surface of a snack food such as a chip or cracker. An Olean/polymeric nanoparticle (PNP) blend can be added into a snack food composition. A silicone/polymeric nanoparticle (PNP) blend can be added to a face cream or a shampoo or a lipstick. A bisabolol/polymeric nanoparticle (PNP) blend can be added to a face cream. A retinoid/polymeric nanoparticle (PNP) blend can be added to a anti-wrinkle cream.

The following non-limiting examples illustrate the practice of the present invention.

### Example 1 ― Preparation of crosslinked polymer nanoparticles useful in personal care applications.

### Preparation of crosslinked polymethylsiloxane diacrylate nanoparticles

Acrylic acid (27 g, 2 eq), hydroquinone (500 ppm) and methane sulfonic acid (3.6 g, 0.2 eq.) is added to dimethicone (100 g) in toluene (34 g). The mixture is refluxed in a Dean-Stark apparatus until all water evolution has ceased, forming the diacrylate functionalized siloxane.

Upon cooling of the diacrylate functionalized polymer formed as in above, Triganox (initiator) 125-C75 (2 g) is added and the mixture gradually added to toluene (570 g) heated at 100°C. Upon completion of the macromonomer feed, the solution is held at temperature for 30 minutes, before addition of 3 chase shots of Triganox 125-C75 (2 g) at 30 minute intervals. The batch is held at temperature for 2.5h before the solvent is removed *in vacuo.*

### Preparation of crosslinked Lauryl methacrylate (LMA) polymer nanoparticles

LMA (114 g), trimethylolpropane triacrylate (12.5 g), and Triganox 125-C75 (1.25 g) is mixed with toluene (31 g) before the mixture is gradually added to toluene (570 g) heated at 100°C. Upon completion of the monomer feed, the solution is held at temperature for 30 minutes, before addition of 3 chase shots of Triganox 125-C75 (1.25 g) at 30 minute intervals. The batch is held at temperature for 2.5 hours before the solvent is removed *in vacuo.*

### Preparation of crosslinked styrene polymer nanoparticles

A plurality of crosslinked emulsion polymer nanoparticles are prepared by a gradual-add polymerization process. A monomer emulsion is made from a mixture of 100 g water, 1.60 g of 28% w/w solids lauryldimethylamine-N-oxide, 55.1 g styrene, and 12.5 g divinyl benzene. A reaction kettle containing 445 g water, 22.2 g of 28% w/w solids lauryldimethylamine-N-oxide (ca. 10 wt%) and 0.37 g ammonium persulfate is heated to 85°C under a nitrogen atmosphere.

The monomer emulsion is fed to the kettle over 90 minutes. The reaction is held at 85°C for 30 minutes after the end of the feed, and then cooled to 65°C. After cooling, 1.33 g of 10% iron sulfate (FeSO₄) is added. After 1 minute, 0.2 g of 70% t-BHP is added and after 2 minutes 0.10 g of 100% isoascorbic acid ("IAA") and the reaction held for 15 minutes. A second chaser system is added in the same sequence and over the same time period. The reaction is then cooled to ambient temperature and filtered through a 400 mesh sieve.

### Example 2 - Preparation of crosslinked PNPs useful in food applications

### Preparation of starch PNPs

Acrylic acid (10 g, 1 eq), hydroquinone (500 ppm) and dicyclohexylcarbodiimide (32 g, 1.1 eq.) is added to amylose acetate (100 g) in dioxane (34 g). The mixture is stirred at 40°C for 8 hours. The solution is filtered and Triganox 125-C75 (2 g) is added to the supernatant before it is gradually added to dioxane (570 g) heated at 80°C. Upon completion of the macromonomer feed, the solution is held at temperature for 30 minutes, before addition of 3 chase shots of Triganox 125-C75 (2g) at 30 minute intervals. The batch is held at temperature for 2.5h before the solvent is removed *in vacuo.*

### Preparation of sugar PNP's

Acrylic acid (10g, 1eq), hydroquinone (500ppm) and dicyclohexylcarbodiimide (32g, 1.1eq.) is added to glucose (100g) in hot dimethylformamide (34g). The mixture is stirred at 60C for 8h. The solution is filtered and cooled before the addition of 4,4'-azobis(4-cyanovaleric acid) (2 g) and sodium hydroxide (0.5 g) to the supernatant. This mixture is gradually added to water (570g) heated at 80°C. Upon completion of the macromonomer feed, the solution is held at temperature for 30 minutes, before addition of 3 chase shots of 4,4'-azobis(4-cyanovaleric acid) (2g in 0.05M NaOH_{(aq)}) at 30 minute intervals. The batch is held at temperature for 2.5 hours before the batch is allowed to cool.

### Example 3 - Skin Care Products Prepared using the Present Invention as in Example 1

Skin care preparations are prepared according to techniques known to those skilled in the art. See also, Balsam & Sagarin.

A representative skin soothing creme is prepared by blending a bisabolol/LMA PNP blend with suitable emollients and thickeners according to methods known to those in the skin care arts.

A representative anti-wrinkle creme can be prepared by blending a retinoid/dimethicone PNP blend with suitable emollients, antioxidant vitamins such as C and E, and thickeners according to methods known to those in the skin care arts.

Additionally, a lipstick can be prepared by blending a pigment/polymethylsiloxane PNP blend with suitable emollients and thickeners according to methods known to those in the skin care arts.

Further, an antibacterial handsoap can be prepared by blending a triclosan/styrene PNP blend with suitable surfactants, emollients, and thickeners according to methods known to those in the skin care arts.

### Example 4 - Snack food product prepared using the present invention as prepared in Example 2.

| **Peanut Butter Spread** | |
|---|---|
| **Ingredient** | **Filling Formula weight percent** |
| Olean®/starch PNP blend *(Procter & Gamble Co.; Cincinnati, OH)* | 31.04 |
| Sugar 12X *(Amalgamated Sugar Co.; Ogden, UT)* | 16.00 |
| Salt *(Morton International, Inc.; Chicago, IL)* | 1.10 |
| Fiber ― soluble *(Fibersol-2, Matsutani Chem. Ind.; Itami-city Hyogo, Japan)* | 5.36 |
| Processed De-fatted (20%) Peanut Flour from U.S. #1 Medium Runner Peanuts *(Cargill Peanut; Dawson, GA)* | 36.43 |
| Vitamin A, D₃, K₁ blend *(Watson Foods Co.; West Haven, CT)* | 0.07 |
| Corn Syrup Solids *(M200, Grain Processing Corp.; Muscatine, IA)* | 10.00 |

### Example 5 - Snack food product prepared using PNPs of the present invention as prepared in Example 2.

| **Chocolate Chip Drop Cookies** | |
|---|---|
| **Ingredient** | **Weight percent** |
| Olean®/starch PNP blend *(Procter & Gamble Co.; Cincinnati, OH)* | 24.28 |
| Whole Egg | 7.90 |
| Chocolate Chips *(Nestle USA; Glendale, CA)* | 14.50 |
| Light Brown Sugar *(Domino Sugar Corp.; New York, NY)* | 13.84 |
| Salt *(Kroger; Cincinnati, OH)* | 0.34 |
| Praline Flavor *(McCormick; Hunt Valley, MD)* | 0.08 |
| All Purpose Flour ― soft wheat *(Siemer Milling Co.; Teutopolis, IL)* | 18.71 |
| Fiber ― soluble *(Fibersol-2, Matsutani Chem. Ind., Itami-city Hyogo, Japan)* | 7.11 |
| Isolated Soy Protein *(Supro® 661, Protein Technologies Intl.; St. Louis, MO)* | 11.70 |
| Sodium Bicarbonate *(Church & Dwight Co.; Princeton, NJ)* | 0.34 |
| Vanilla Flavor *(Nielsen-Massey Vanilla, Inc.; Waukegan, IL)* | 1.20 |

### Example 6 ― Snack food product prepared using PNPs of the present invention as prepared in Example 2.

A barbecue flavor/starch PNP blend can be sprayed onto the surface of a snack food such as a chip or cracker according to methods known to those in the food industry.

### Example 7 - Cereal product prepared using PNPs of the present invention as prepared in Example 2.

A vitamin E/sugar PNP blend can be sprayed onto the surface of a flaked cereal according to methods known to those in the food industry.

## Claims

1. Crosslinked polymeric nanoparticles having a diameter of 1-10 nm comprising skin care ingredients.

2. Crosslinked polymeric nanoparticles according to Claim 1, formed from synthetic materials.

3. Crosslinked polymeric nanoparticles according to Claim 1, formed from natural materials.

4. Crosslinked polymeric nanoparticles according to Claim 2, wherein said synthetic materials are unsaturated and multiethylenically unsaturated monomers.

5. Crosslinked polymeric nanoparticles having a diameter of 1-10 nm comprising food ingredients.

6. Crosslinked polymeric nanoparticles according to Claim 5, formed from natural materials.

7. Crosslinked polymeric nanoparticles according to Claim 5, formed from synthetic materials.

8. Crosslinked polymeric nanoparticles according to Claim 6, wherein said natural materials are sugar and starch.

9. A skin care composition comprising a skin care ingredient/polymeric nanoparticle blend.

10. A snack food composition comprising a food ingredient/polymeric nanoparticle blend.
